# EUROPÄISCHE PATENTSCHRIFT

(11) **EP 2 069 282 B1**
(45) Veröffentlichungstag und Bekanntmachung des Hinweises auf die Patenterteilung: **11.07.2012**
(21) Anmeldenummer: 07786443.7
(22) Anmeldetag: 31.07.2007
(51) Int. Cl.: C07C 67/03, C07C 69/24, C10L 1/18, C10L 1/02, C11C 3/10, C11C 3/00

(54) **VERFAHREN ZUR KONTINUIERLICHEN HERSTELLUNG VON FETTSÄURE-METHYLESTER ODER FETTSÄURE-ETHYLESTER**
PROCESS FOR CONTINUOUSLY PREPARING FATTY ACID METHYL ESTERS OR FATTY ACID ETHYL ESTERS
PROCEDE DE PRODUCTION CONTINUE D'ESTER METHYLIQUE D'ACIDE GRAS OU D'ESTER ETHYLIQUE D'ACIDE GRAS

(30) Priorität: 21.09.2006 DE 102006044467
(43) Veröffentlichungstag der Anmeldung: 17.06.2009
(73) Patentinhaber: Lurgi GmbH, 60439 Frankfurt am Main (DE)
(72) Erfinder: SAFT, Helmut, 61194 Niddatal (DE); HOHMANN, Klaus, 65719 Hofheim (DE); BÖNSCH, Rudolph, 55299 Nackenheim (DE)
(86) Internationale Anmeldenummer: PCT/EP2007/006745
(87) Internationale Veröffentlichungsnummer: WO 2008/034485

(56) Entgegenhaltungen:
- EP-A- 0 523 767
- WO-A-2006/088254

## Beschreibung

Die Erfindung betrifft ein Verfahren zur kontinuierlichen Herstellung von Fettsäure-Methylester (FAME) oder Fettsäure-Ethylester (FAEE), insbesondere für Diesel-Brennkraftmaschinen, aus pflanzlichen und/oder tierischen Fetten und/oder Ölen durch Umesterung der in den Fetten und Ölen enthaltenen Triglyceride mit Methanol oder Ethanol in Gegenwart eines alkalischen Katalysators in flüssiger Phase bei einer Temperatur von 30 bis 90° C und bei Atmosphärendruck in mindestens zwei nacheinander durchströmten, jeweils aus einem Rührkesselreaktor mit nachgeschaltetem Abscheider bestehenden Reaktionsstufen, indem in den Rührkesselreaktoren eine FAME oder FAEE enthaltende leichte Phase und eine Glyzerin enthaltende schwere Phase erzeugt werden, die FAME oder FAEE enthaltende leichte Phase und die Glyzerin enthaltende schwere Phase in dem dem jeweiligen Rührkesselreaktor nachgeschalteten Abscheider voneinander getrennt werden, die FAME oder FAEE enthaltende Phase dem Rührkesselreaktor der nächst folgenden Reaktionsstufe zugeführt wird, die aus dem Abscheider der zweiten bis letzten Reaktionsstufe abgezogene Glyzerin enthaltende Phase in den Rührkesselreaktor der ersten Reaktionsstufe rückgeführt wird, der aus dem Abscheider der letzten Reaktionsstufe abgezogene Roh-FAME oder Roh-FAEE nach einer saueren Wasserwäsche in einen Abscheider überführt und der aus diesem Abscheider abgezogene FAME oder FAEE getrocknet wird.

Das vorstehend beschriebene, aus der DE-A-4123928 bekannte Verfahren zur Erzeugung von Fettsäure-Methylester (FAME) oder Fettsäure-Äthylester (FAEE) und Glyzerin durch Umesterung von Ölen oder Fetten mindestens zwei Reaktionsstufen betrieben, wobei jede Reaktionsstufe einen Mischreaktor mit einen nachgeschalteten Abscheider zur physikalischen Trennung in eine leichte esterreiche Phase und eine schwere glyzerinreiche Phase aufweist. Dem Mischreaktor der ersten Umsetzungsstufe wird Öl oder Fett sowie Methanol oder Äthanol und Katalysator und dem Mischreaktor der zweiten und jeder weiteren Umsetzungsstufe Methanol oder Äthanol und Katalysator sowie die in der jeweils vorausgehenden Reaktionsstufe gewonnene leichte esterreiche Phase zugeführt. Die im Abscheider der zweiten bis letzten Reaktionsstufe abgetrennte schwere glyzerinreiche Phase wird mindestens teilweise in den Mischreaktor der ersten Reaktionsstufe rückgeführt. Aus der in der letzten Reaktionsstufe abgetrennten leichten esterreichen Phase wird FAME oder FAEE durch mindestens eine weitere Separationsbehandlung gewonnen. Dieses Verfahren, mit dem sich relativ große Materialdurchsätze erzielen lassen, ist auf relativ einfache und kostengünstige Weise durchführbar.

Die Menge des insgesamt den Mischreaktoren zugeführten Methanols oder Ethanols beträgt des 1- bis 3-fache der stöchiometrisch notwendigen Menge. Als alkalische Katalysatoren werden vorzugsweise NaOH, KOH, CH₃ONa oder C₂H₅ONa, im allgemeinen in einer Konzentration von 0.1 bis 1.0 Gew. %, bezogen auf die Menge des umzusetzenden Fetts oder Öls, verwendet.

Beim Betrieb des Verfahrens ist es zweckmäßig dem Mischreaktor der ersten Reaktionsstufe 20 bis 60 %, vorzugsweise 35 bis 45 %, des gesamten Methanols oder Ethanols sowie 20 bis 60 %, vorzugsweise 30 bis 50 % des gesamten alkalischen Katalysators zuzuführen. Der Rest des Methanols oder Ethanols und des Katalysators wird dem Mischreaktor der zweiten Reaktionsstufe aufgegeben. Hierbei bleiben die kleinen Mengen unberücksichtigt, die durch Rückführung der glyzerinreichen Phase aus dem Abscheider der zweiten Reaktionsstufe in den Mischreaktor der ersten Reaktionsstufe gelangen.

Von der aus dem Abscheider der ersten Reaktionsstufe abgezogenen schweren glyzerinreichen Phase wird das Methanol oder Ethanol vom Glyzerin thermisch abgetrennt und in den Mischreaktor der ersten Reaktionsstufe rückgeführt. Die aus dem Abscheider der letzten Reaktionsstufe abgeführte leichte esterreiche Phase wird nach einer Extraktion mit Wasser einer Trennung unter Einwirkung der Zentrifugalkraft unterworfen und in FAME oder FAEE mit einem Wassergehalt von max. 2.0 Gew. % und in eine wässrige Methanol oder Äthanol und Glyzerin enthaltende Phase getrennt. Um den Wassergehalt des FAME oder FAEE zu senken, besteht die Möglichkeit, den FAME oder FAEE in einem Wärmetauscher zu erhitzen und anschließend eine Trocknung durchzuführen. Die wässrige Methanol oder Ethanol und Glyzerin enthaltende von Methanol oder Ethanol nahezu freie Phase wird der aus dem Abscheider der ersten Reaktionsstufe ausgetragenen schweren glyzerinreichen Phase vor deren thermischen Trennung zugesetzt.

Bei der Zweiphasentrennung der Umesterungsprodukte in den den Mischreaktoren nachgeordneten Abscheidern in eine leichte esterreiche Phase und eine schwere glyzerinreiche Phase bleiben in der schweren glyzerinreichen Phase bis zu 3 Gew. % Ester zurück. Darüber hinaus werden bei der sauren Wasserwäsche der aus der letzten Reaktionsstufe gewonnenen esterreichen Phase bis zu 2 Gew. % Ester ausgewaschen, die dann nach der Abtrennung des FAME oder FAEE in der wässrigen, Methanol oder Ethanol, Glyzerin und ungespaltene Seifen enthaltenden Phase enthalten sind. Bei der thermischen Trennung der Mischung aus der glyzerinreichen Phase der ersten Reaktionsstufe und Methanol oder Ethanol und Glyzerin der wässrigen Phase in Methanol oder Ethanol einerseits und Roh-Glyzerin andererseits verbleibt Ester in dem Roh-Glyzerin.

Es ist die Aufgabe der vorliegenden Erfindung das eingangs beschriebene Verfahren so auszubilden, dass die Gesamtausbeute an FAME oder FAEE erhöht und damit die Wirtschaftlichkeit des Verfahrens erkennbar verbessert wird.

Diese Aufgabe wird dadurch gelöst, dass die nach der Abtrennung von FAME oder FAEE nach der saueren Wasserwäsche anfallende 0,5 bis 2 Gew. % Glyzerin, 2 bis 8 Gew. % Methanol oder Ethanol, 0,05 bis 0,5 Gew. ungespaltene Seifen und 0,05 bis 0,5 Gew. % FAME oder FAEE enthaltende wässrige Phase mit den aus den Abscheidern der ersten bis vorletzten Reaktionsstufe abgezogenen Glyzerin und 0,5 bis 3 Gew. % FAME oder FAEE enthaltenden schweren Phasen durchmischt und dann einem Abscheider zugeführt wird, in dem eine Trennung in eine FAME oder FAEE enthaltende Phase und eine Glyzerin enthaltende Phase durchgeführt wird.

Eine Weiterbildung dieser Maßnahme besteht darin, dass die Glyzerin enthaltende Phase thermisch in Roh-Glyzerin und Methanol oder Ethanol getrennt und Methanol oder Ethanol und der aus dem Abscheider abgezogene FAME oder FAEE in den Rührkesselreaktor der ersten Reaktionsstufe rückgeführt werden.

Ausgestaltet ist die Erfindung dadurch, dass die 0,5 bis 2 Gew. % Glyzerin, 2 bis 8 Gew. Methanol oder Ethanol, 0,05 bis 0,5 Gew. % ungespaltene Seifen und 0,05 bis 0,5 Gew. % FAME oder FAEE enthaltende wässrige Phase mit den Glyzerin und 0.5 bis zu 3 Gew. % FAME oder FAEE enthaltenden schweren Phasen bei einem pH-Wert von 0,5 bis < 6, vorzugsweise einem pH-Wert von 1 bis 4, und einer Temperatur von 40 bis 60° C, vorzugsweise einer Temperatur von 40 bis 50° C, für die Dauer von 15 bis 60 min, vorzugsweise für die Dauer von 20 bis 45 min, durchmischt und die Mischung auf eine Temperatur von 25 bis 35° C abgekühlt wird, infolgedessen der FAME oder FEE auf der Mischung aufschwimmt und problemlos abgezogen werden kann.

Zweckmäßigerweise wird die saure Wasserwäsche des Roh-FAME oder Roh-FAEE mit einer schwachen Säure, vorzugsweise mit 1 bis 5 %iger Salzsäure, Schwefelsäure, Phosphorsäure oder Zitronensäure in einem Intensivmischer durchgeführt.

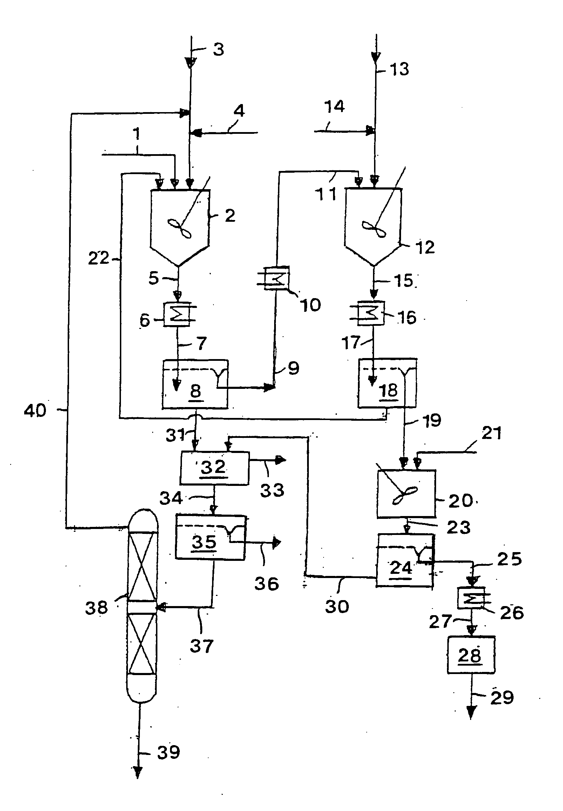

Die Erfindung wird nachfolgend durch ein Ausführungsbeispiel und an Hand eines in der Zeichnung dargestellten Verfahrensfließbilds näher erläutert.

Über Leitung (1) werden dem Rührkesselreaktor (2) der ersten Reaktionsstufe 1000 kg/h durch Entschleimen, Bleichen und Entsäuem vorbehandeltes Rapsöl, über Leitung (3) 74 kg/h wasserfreies Methanol und über Leitung (4) 2,4 kg/h als Katalysator dienendes Natriummethylat (CH₃ONa) zugeführt. Bei der Herstellung von FAEE wird Natriumethylat (C₂H₅ONa) als Katalysator eingesetzt. Diese Komponenten werden bei einer Temperatur von 75° C und bei Atmosphärendruck in dem Rührkesselreaktor (2) intensiv durchmischt. Der Rührkesselreaktor (2) kann bekanntermaßen aus mehreren hintereinander angeordneten Mischkammem bestehen. Im wesentlichen besteht das Rapsöl aus Glyzerin als Grundträger mit drei langen an ihm angelagerten Fettsäure-Ketten (C₁₈-Ketten); diese reagieren bei der Umesterung mit Methanol, wobei der Katalysator die Reaktion unterstützt. Bei der Umesterung wird das langkettige, verzweigte Molekül gegen drei einzelne, kürzere Moleküle ausgetauscht. Öle und Fette bestehen zu 97 % aus Glyzerin als Grundträger und daran angelagerten Fettsäure-Ketten mit 16 bis 22 C-Atomen, die sich unter Abspaltung von Wasser mit dem Glyzerin verbunden haben; derartige Verbindungen werden als Triglyceride bezeichnet. Der Rest sind freie Fettsäuren, Vitamine, Wasser, Phosphor-Verbindungen und geringe Schwefel-Anteile. Aus dem Rührkesselreaktor (2) strömt das Umesterungsprodukt durch Leitung (5) über den Wärmetauscher (6), wird in diesem auf eine Temperatur von 60° C abgekühlt und danach über Leitung (7) dem Abscheider (8) aufgegeben, in dem eine Trennung in eine FAME enthaltende leichte Phase und eine Glyzerin enthaltende schwere Phase stattfindet. Die über Leitung (9) aus dem Abscheider (8) abgezogene FAME enthaltende leichte Phase wird über den Wärmetauscher (10), in dem die Temperatur des Umesterungsprodukt auf 45° C erniedrigt wird, geleitet und anschließend über Leitung (11) dem Rührkesselreaktor (12) der zweiten Reaktionsstufe zugeführt. Dabei werden dem Umesterungsprodukt über Leitung (13) 118 kg/h Methanol und über Leitung (14) 3,6 kg/h CH₃ONa als Katalysator zugesetzt und die Komponenten intensiv bei einer Temperatur von 60° C und bei Atmosphärendruck durchmischt. Das über Leitung (15) aus dem Rührkesselreaktor (12) abströmende Umesterungsprodukt wird in dem nachfolgenden Wärmeaustauscher (16) auf eine Temperatur von 35° C abgekühlt und dann über Leitung (17) dem Abscheider (18) aufgegeben, aus dem über Leitung (19) Roh-FAME, in dem wenigstens 99 % der im Rapsöl enthaltenen Fettsäuren in Form von FAME vorliegen, abgezogen und einem Mixer (20) zugeführt. In dem Mixer (20) werden unter Zusatz von über Leitung (21) zufließender 3 %iger Salzsäure bei einem pH-Wert von 6 bis 7 die in dem Roh-FAME enthaltenen Restseifen gespalten und der Katalysator sowie die Phosphor-Verbindungen zerstört und die wasserlöslichen Substanzen, im wesentlichen Methanol und Glyzerin extrahiert. Die aus dem Abscheider (18) über Leitung (22) abgezogene Glyzerin enthaltende schwere Phase wird in den Rührkesselreaktor (1) der ersten Reaktionsstufe rückgeführt. Die über Leitung (23) aus dem Mixer (20) abströmenden FAME enthaltenden Phase wird in einem nachgeschalteten Abscheider (24) in FAME mit einem Wassergehalt von weniger als 2 Gew. % und in eine im wesentlichen 1,5 Gew. Glyzerin, 4,5 Gew. % Methanol, 0,3 Gew. % ungespaltene Seifen und 0,25 Gew. % FAME enthaltende wässrige Phase getrennt. Der über Leitung (25) aus dem Abscheider (24) abgeführte FAME wird nach einer in dem nachgeordneten Wärmetauscher (26) erfolgenden Vorwärmung auf eine Temperatur von 95° C über Leitung (27) einem Vakuumtrockner (28) zugeführt, in dem das in dem FAME enthaltene Wasser abgetrieben wird. Über Leitung (29) werden aus dem Vakuumtrockner (28) 1001 kg/h FAME ausgeleitet, der bei Prüfung in Übereinstimmung mit angegebenen Prüfverfahren die in der europäischen Norm für Biodiesel (DIN EN 14214) gestellten Anforderungen erfüllt.

Die über Leitung (30) den Abscheider (24) verlassende wässrige Phase der vorstehend angeführten Zusammensetzung und die über Leitung (31) aus dem Abscheider (8) der ersten Reaktionsstufe abströmende Glyzerin und 2.5 Gew. % FAME enthaltende schwere Phase werden in den Rührbehälter (32) geleitet, in dem diese für die Dauer von 30 min intensiv durchmischt, auf eine Temperatur von 24 °C erwärmt und der pH-Wert durch den Zusatz von Salzsäure auf 2 eingestellt wird. Der auf der Oberfläche der Mischung aufschwimmende FAME wird über Leitung (33) aus dem Rührbehälter (32) ausgeleitet und kann in den Rührkessel (2) der ersten Reaktionsstufe rückgeführt werden. Die neben Glyzerin noch Methanol und FAME enthaltende Phase wird über Leitung (34) aus dem Rührbehälter (32) abgeleitet und dem Abscheider (35) aufgegeben, in dem der FAME bei einer Temperatur von 20°C und bei einem Konzentrationsgefälle in der Glyzerin enthaltenden Phase von 7 % nahezu vollständig abgeschieden und 0,5 kg/h FAME über Leitung (36) aus dem Abscheider (35) abgezogen werden. Die verbleibende nahezu nur noch Glyzerin und Methanol enthaltende wässrige Phase strömt über Leitung (37) zu der Rektifikationskolonne (38), in der bei einer Sumpftemperatur von 110 °C und bei Atmosphärendruck eine Trennung in eine Glyzerinphase und eine Methanolphase erfolgt. Aus dem Sumpf der Rektifikationskolonne (38) werden über Leitung (39) 1,5 %iges Glyzerin und am Kopf über Leitung (40) 99,9 %iges Methanol abgezogen. Das Methanol wird in den in Leitung (3) strömenden dem Rührkesselreaktor (2) der ersten Reaktionsstufe zugeführten Methanolstrom eingespeist und die wässrige Glyzerinphase einer Reinigung zugeführt.

Durch die erfindungsgemäßen Maßnahmen gelingt es, die Gesamtausbeute an FAME und FAEE um bis zu 2 % zu erhöhen und somit die Gesamtwirtschaftlichkeit des Verfahrens deutlich zu verbessern.

## Patentansprüche

1. Verfahren zur kontinuierlichen Herstellung von Fettsäure-Methylester (FAME) oder Fettsäure-Ethylester (FAEE), insbesondere für Diesel-Brennkraftmaschinen, aus pflanzlichen und/oder tierischen Fetten und/oder Ölen durch Umesterung der in den Fetten und Ölen enthaltenen Triglyceride mit Methanol oder Äthanol in Gegenwart eines alkalischen Katalysators in flüssiger Phase bei einer Temperatur von 30 bis 90° C und bei Atmosphärendruck in mindestens zwei nacheinander durchströmten, jeweils aus einem Rührkesselreaktor (2, 12) mit nachgeschaltetem Abscheider (8, 18) bestehenden Reaktionsstufen, indem in den Rührkesselreaktoren eine FAME oder FAEE enthaltende leichte Phase und eine Glyzerin enthaltende schwere Phase erzeugt werden, die FAME oder FAEE enthaltende leichte Phase und die Glyzerin enthaltende schwere Phase in dem dem jeweiligen Rührkesselreaktor nachgeschalteten Abscheider voneinander getrennt werden, die FAME oder FAEE enthaltende Phase dem Rührkesselreaktor der nächst folgenden Reaktionsstufe zugeführt wird, die aus dem Abscheider der zweiten bis letzten Reaktionsstufe abgezogene Glyzerin enthaltende Phase in den Rührkesselreaktor der ersten Reaktionsstufe rückgeführt wird, der aus dem Abscheider der letzten Reaktionsstufe abgezogene Roh-FAME oder Roh-FAEE nach einer saueren Wasserwäsche in einen Abscheider (24) überführt und der aus diesem Abscheider abgezogene FAME oder FAEE getrocknet wird, **dadurch gekennzeichnet, dass** die aus dem Abscheider (24) abgeführte 0,5 bis 2 Gew. % Glycerin, 2 bis 8 Gew. % Methanol oder Ethanol, 0.05 bis 0,5 Gew. % ungespaltene Seifen und 0,05 bis 0,5 Gew. % FAME oder FAEE enthaltende wässrige Phase mit den aus den Abscheidern (8) der ersten bis vorletzten Reaktionsstufe abgezogenen Glyzerin und 0,5 bis 3 % FAME oder FAEE enthaltenden schweren Phasen durchmischt und dann einem Abscheider (35) zugeführt wird, in dem eine Trennung in eine FAME oder FAEE enthaltende Phase und in eine Glyzerin enthaltende Phase erfolgt.

2. Verfahren nach Anspruch 1, **dadurch gekennzeichnet, dass** die aus dem Abscheider (35) abgezogene Glyzerin enthaltende Phase thermisch in Roh-Glyzerin und Methanol oder Ethanol getrennt und Methanol oder Ethanol und der aus dem Abscheider (35) abgezogene FAME oder FAEE in den Rührkesselreaktor (2) der ersten Reaktionsstufe rückgeführt werden.

3. Verfahren nach Anspruch 1, **dadurch gekennzeichnet, dass** die wässrige 0,5 bis 2 Gew. % Glyzerin, 2 bis 8 Gew. % Methanol, 0,05 bis 0,5 Gew. % ungespaltene
Seifen und 0,05 bis 0,5 Gew. % FAME oder FAEE enthaltende Phase und Glyzerin mit 0,5 bis 3 Gew. % FAME oder FAEE enthaltenden Phase bei einem pH-Wert von 0,5 bis < 6 und einer Temperatur von 40 bis 60°C für die Dauer von 15 bis 60 min durchmischt und die Mischung auf eine Temperatur von 25 bis 35° C abgekühlt wird.

4. Verfahren nach Anspruch 3, **dadurch gekennzeichnet, dass** die Durchmischung bei einem pH-Wert von 1 bis 4 und einer Temperatur von 40 bis 50°C für die Dauer von 20 bis 45 min erfolgt.

5. Verfahren nach einem der Ansprüche 1 bis 4, **dadurch gekennzeichnet, dass** die saure Wasserwäsche des Roh-FAME oder Roh-FAEE mit einer schwachen Säure, vorzugsweise mit 1 bis 5 %iger Salzsäure, Schwefelsäure, Phosphorsäure oder Zitronensäure in einem Intensivmischer (20) durchgeführt wird

## Claims

1. A process for the continuous production of fatty acid methyl ester (FAME) or fatty acid ethyl ester (FAEE), in particular for Diesel combustion engines, from vegetable and/or animal fats and/or oils by transesterification of the triglycerides contained in the fats and oils with methanol or ethanol in the presence of an alkaline catalyst in liquid phase at a temperature of 30 to 90°C and at atmospheric pressure in at least two reaction stages traversed in succession, which each consist of a stirred-tank reactor (2, 12) with downstream separator (8, 18), in that a light phase containing FAME or FAEE and a heavy phase containing glycerol are generated in the stirred-tank reactors, the light phase containing FAME or FAEE and the heavy phase containing glycerol are separated from each other in the separator downstream of the respective stirred-tank reactor, the phase containing FAME or FAEE is supplied to the stirred-tank reactor of the next succeeding reaction stage, the glycerol-containing phase withdrawn from the separator of the second to last reaction stages is recirculated into the stirred-tank reactor of the first reaction stage, the crude FAME or crude FAEE withdrawn from the separator of the last reaction stage is transferred into a separator (24) after acid water washing, and the FAME or FAEE withdrawn from this separator is dried, **characterized in that** the aqueous phase containing 0.5 to 2 wt-% of glycerol, 2 to 8 wt-% of methanol or ethanol, 0.05 to 0.5 wt-% of undissociated soaps and 0.05 to 0.5 wt-% of FAME or FAEE, which is discharged from the separator (24), is thoroughly mixed with the heavy phases withdrawn from the separators (8) of the first to penultimate reaction stages, which contain glycerol and 0.5 to 3 wt-% of FAME or FAEE, and then is supplied to a separator (35), in which a separation into a phase containing FAME or FAEE and a phase containing glycerol is effected.

2. The process according to claim 1, **characterized in that** the glycerol-containing phase withdrawn from the separator (35) is thermally separated into crude glycerol and methanol or ethanol, and methanol or ethanol and the FAME or FAEE withdrawn from the separator (35) are recirculated into the stirred-tank reactor (2) of the first reaction stage.

3. The process according to claim 1, **characterized in that** the aqueous phase containing 0.5 to 2 wt-% of glycerol, 2 to 8 wt-% of methanol, 0.05 to 0.5 wt-% of undissociated soaps and 0.05 to 0.5 wt-% of FAME or FAEE and the phase containing glycerol with 0.5 to 3 wt-% of FAME or FAEE at a pH value of 0.5 to <6 and a temperature of 40 to 60°C are thoroughly mixed for a period of 15 to 60 min, and the mixture is cooled to a temperature of 25 to 35°C.

4. The process according to claim 3, **characterized in that** mixing is effected at a pH value of 1 to 4 and a temperature of 40 to 50°C for a period of 20 to 45 min.

5. The process according to any of claims 1 to 4, **characterized in that** the acid water washing of the crude FAME or crude FAEE is performed with a weak acid, preferably with 1 to 5% hydrochloric acid, sulfuric acid phosphoric acid or citric acid in an intensive mixer (20).

## Revendications

1. Procédé de préparation en continu d'ester méthylique d'acide gras (FAME) ou d'ester éthylique d'acide gras (FAEE), notamment pour des moteurs à combustion interne diesel, à partir de matières grasses et/ou d'huiles végétales et/ou animales, par transestérification des triglycérides contenus dans les matières grasses et les huiles par du méthanol ou par de l'éthanol, en présence d'un catalyseur alcalin en phase liquide, à une température de 30 à 90° C et sous la pression atmosphérique, dans au moins deux étages de réaction parcourus l'un après l'autre et constitués respectivement d'un réacteur (2, 12) à chaudron de brassage ayant un séparateur (8, 18) en aval, dans lequel on produit dans les réacteurs à chaudron de brassage une phase légère contenant du FAME ou du FAEE et une phase lourde contenant de la glycérine, on sépare l'une de l'autre la phase légère contenant du FAME ou du FAEE et la phase lourde contenant de la glycérine dans le séparateur en aval de chaque réacteur à chaudron de brassage on envoie la phase contenant du FAME ou du FAEE au réacteur à chaudron de brassage, de l'étage de réaction immédiatement suivant, on retourne la phase contenant de la glycérine soutirée du séparateur du deuxième au dernier étages de réaction au réacteur à chaudron de brassage du premier étage de réaction, on transvase le FAME brut ou le FAEE brut soutiré du séparateur du dernier étage, de réaction après un lavage à l'eau acide, à un séparateur (24) et on sèche le FAME ou le FAEE soutiré du séparateur, **caractérisé en ce que** l'on mélange la phase aqueuse soutirée du séparateur (24) et contenant de 0,05 à 2 % en poids de glycérine, de 2 à 8 % en poids de méthanol ou d'éthanol, de 0,05 à 0,5% en poids de savons non décomposés et de 0,05 à 0,5 % en poids de FAME ou de FAEE à la glycérine soutirée des séparateurs (8) des premier à avant dernier étages de réaction et aux phases lourdes contenant de 0,5 à 3% de FAME ou de FAEE et on les envoie ensuite à un séparateur (35) dans lequel s'effectue une séparation en une phase contenant du FAME ou de FAEE et en une phase contenant de la glycérine.

2. Procédé suivant la revendication 1, **caractérisé en ce que** l'on sépare thermiquement la phase contenant de la glycérine soutirée du séparateur (35) en de la glycérine brute et en du méthanol ou de l'éthanol et on retourne du méthanol ou de l'éthanol et le FAME ou le FAEE soutiré du séparateur (35) au réacteur (2) à chaudron de brassage du premier étage de réaction.

3. Procédé suivant la revendication 1, **caractérisé en ce que** l'on mélange la phase aqueuse contenant de 0,5 à 2% en poids de glycérine, de 2 à 8% en poids de méthanol, de 0,05 à 0,5 % en poids de savons non décomposés et de 0,05 à 0,5% en poids de FAME ou de FAEE et de la glycérine à une phase contenant de 0,5 à 3% en poids de FAME ou de FAEE, à un pH allant de 0,5 à < 6 et à une température de 40 à 60° C, pendant une durée de 15 à 60 minutes et on refroidit le mélange jusqu'à une température de 25-à 35° C.

4. Procédé suivant la revendication 3, **caractérisé en ce que** l'on effectue le mélange un pH de 1 à 4 et à une température de 40 à 50° C pendant une durée de 20 à 45 minutes.

5. Procédé suivant l'une des revendications 1 à 4, **caractérisé en ce que** l'on effectue le lavage à l'eau acide du FAME brut ou du FAEE brut par un acide faible, de préférence par de l'acide chlorhydrique, de l'acide sulfurique, de l'acide phosphorique ou de l'acide citrique à 1 à 5 % dans un mélangeur (20) intensif.
